(19) Europäisches Patentamt
European Patent Office
Office européen des brevets

(11) EP 1 922 074 B1

(12) **EUROPEAN PATENT SPECIFICATION**

(45) Date of publication and mention of the grant of the patent:
**17.02.2010 Bulletin 2010/07**

(21) Application number: **06769626.0**

(22) Date of filing: **24.08.2006**

(51) Int Cl.:
***A61K 31/58*** (2006.01) ***A61K 31/167*** (2006.01)
***A61K 31/4375*** (2006.01) ***A61K 31/4468*** (2006.01)
***A61P 11/00*** (2006.01) ***A61P 11/06*** (2006.01)
***A61P 11/08*** (2006.01)

(86) International application number:
**PCT/SE2006/000970**

(87) International publication number:
**WO 2007/024182 (01.03.2007 Gazette 2007/09)**

(54) **A COMBINATION OF COMPOUNDS, WHICH CAN BE USED IN THE TREATMENT OF RESPIRATORY DISEASES, ESPECIALLY CHRONIC OBSTRUCTIVE PULMONARY DISEASE (COPD) AND ASTHMA.**

KOMBINATION VON VERBINDUNGEN, DIE BEI DER BEHANDLUNG VON ATEMWEGSERKRANKUNGEN VERWENDET WERDEN KÖNNEN, INSBESONDERE BEI CHRONISCH OBSTRUKTIVER LUNGENERKRANKUNG (COPD) UND ASTHMA

COMBINAISON DE COMPOSÉS POUVANT ÊTRE EMPLOYÉE DANS LE TRAITEMENT DE MALADIES RESPIRATOIRES, EN PARTICULIER DE BRONCHO-PNEUMOPATHIE CHRONIQUE OBSTRUCTIVE (BPCO) ET D'ASTHME

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **26.08.2005 SE 0501896**
**01.06.2006 SE 0601220**

(43) Date of publication of application:
**21.05.2008 Bulletin 2008/21**

(73) Proprietor: **AstraZeneca AB**
**151 85 Södertälje (SE)**

(72) Inventors:
- **ERIKSSON, Tomas**
**S-221 87 Lund (SE)**
- **HANSSON, Johan**
**S-221 87 Lund (SE)**

(74) Representative: **Wahlström, Stig Christer Gunnar et al**
**AstraZeneca**
**Global Intellectual Property**
**S-151 85 Södertälje (SE)**

(56) References cited:
**WO-A-00/44408** **WO-A1-02/088167**
**WO-A1-03/051839** **WO-A1-03/082292**
**WO-A2-01/98270** **WO-A2-02/45703**
**WO-A2-2005/010154** **WO-A2-2005/025555**
**US-A- 3 929 768** **US-A- 3 994 974**

- **ROLLAND C ET AL: "G-Protein-Coupled Receptor Affinity Prediction Based on the Use of a profiling Dataset: QSAR Design, Synthesis, and Experimental Validation" JOURNAL OF MEDICINAL CHEMISTRY, AMERICAN CHEMICAL SOCIETY. WASHINGTON.; US, vol. 48, no. 21, 22 September 2005 (2005-09-22), pages 6563-6574, XP002439868 ISSN: 0022-2623**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 922 074 B1

## Description

The present invention relates to combinations of pharmaceutically active substances for use in the treatment of respiratory disorders, especially chronic obstructive pulmonary disease (COPD) and asthma.

BACKGROUND OF THE INVENTION

The essential function of the lungs requires a fragile structure with enormous exposure to the environment, including pollutants, microbes, allergens, and carcinogens. Host factors, resulting from interactions of lifestyle choices and genetic composition, influence the response to this exposure. Damage or infection to the lungs can give rise to a wide range of diseases of the respiratory system (or respiratory diseases). A number of these diseases are of great public health importance. Respiratory diseases include Acute Lung Injury, Acute Respiratory Distress Syndrome (ARDS), occupational lung disease, lung cancer, tuberculosis, fibrosis, pneumoconiosis, pneumonia, emphysema, Chronic Obstructive Pulmonary Disease (COPD) and asthma.

Among the most common respiratory diseases is asthma. Asthma is generally defined as an inflammatory disorder of the airways with clinical symptoms arising from intermittent airflow obstruction. It is characterised clinically by paroxysms of wheezing, dyspnea and cough. It is a chronic disabling disorder that appears to be increasing in prevalence and severity. It is estimated that 15% of children and 5% of adults in the population of developed countries suffer from asthma. Therapy should therefore be aimed at controlling symptoms so that normal life is possible and at the same time provide basis for treating the underlying inflammation.

COPD is a term which refers to a large group of lung diseases which can interfere with normal breathing. Current clinical guidelines define COPD as a disease state characterized by airflow limitation that is not fully reversible. The airflow limitation is usually both progressive and associated with an abnormal inflammatory response of the lungs to noxious particles and gases. The most important contributory source of such particles and gases, at least in the western world, is tobacco smoke. COPD patients have a variety of symptoms, including cough, shortness of breath, and excessive production of sputum; such symptoms arise from dysfunction of a number of cellular compartments, including neutrophils, macrophages, and epithelial cells. The two most important conditions covered by COPD are chronic bronchitis and emphysema.

Chronic bronchitis is a long-standing inflammation of the bronchi which causes increased production of mucous and other changes. The patients' symptoms are cough and expectoration of sputum. Chronic bronchitis can lead to more frequent and severe respiratory infections, narrowing and plugging of the bronchi, difficult breathing and disability.

Emphysema is a chronic lung disease which affects the alveoli and/or the ends of the smallest bronchi. The lung loses its elasticity and therefore these areas of the lungs become enlarged. These enlarged areas trap stale air and do not effectively exchange it with fresh air. This results in difficult breathing and may result in insufficient oxygen being delivered to the blood. The predominant symptom in patients with emphysema is shortness of breath.

Therapeutic agents used in the treatment of respiratory diseases include glucocorticosteroids. Glucocorticosteroids (also known as corticosteroids or glucocorticoids) are potent anti-inflammatory agents. Whilst their exact mechanism of action is not clear, the end result of glucocorticosteroid treatment is a decrease in the number, activity and movement of inflammatory cells into the bronchial submucosa, leading to decreased airway responsiveness. Glucoorticosteroids may also cause reduced shedding of bronchial epithelial lining, vascular permeability, and mucus secretion.

Whilst gluocorticosteroid treatment can yield important benefits, the efficacy of these agents is often far from satisfactory, particularly in COPD. Moreover, whilst the use of steroids may lead to therapeutic effects, it is desirable to be able to use steroids in low doses to minimise the occurrence and severity of undesirable side effects that may be associated with regular administration. Recent studies have also highlighted the problem of the acquisition of steroid resistance amongst patients suffering from respiratory diseases. For example, cigarette smokers with asthma have been found to be insensitive to short term inhaled corticosteroid therapy, but the disparity of the response between smokers and non-smokers appears to be reduced with high dose inhaled corticosteroid (Tomlinson et al., Thorax 2005;60: 282-287). Hence there is a pressing medical need for new therapies against respiratory diseases such as COPD and asthma, in particular for therapies with disease modifying potential.

WQ01/98273 and WO03/051839 describe compounds having activity as pharmaceuticals, in particular as modulators of chemokine receptor (especially MIP-1$\alpha$ chemokine receptor), salts thereof and pharmaceutical formulations, and their potential use in treating various diseases.

The MIP-1$\alpha$ chemokine receptor CCR1 (chemokine receptor 1) is highly expressed in tissues affected in different autoimmune, inflammatory, proliferative, hyperproliferative and immunologically mediated diseases e.g. asthma and chronic obstructive pulmonary disease. Moreover, inflammatory cells (e.g. neutrophils and monocytes/macrophages) contribute to the pathogenesis of respiratory diseases such as COPD by secretion of proteolytic enzymes, oxidants and pharmacologic mediators. These cells are dependent on the function of CCR1 for recruitment and activation in lung tissues.

Surprisingly, it has now been found that an unexpectedly beneficial therapeutic effect may be observed in the treatment of respiratory diseases if a CCR1 receptor antagonist is used in combination with a glucocorticosteroid. For example, the combination according to the invention is considered to be particularly effective in reducing inflammatory cell influx into the lung. The beneficial effect may be observed when the two active substances are administered simultaneously (either in a single pharmaceutical preparation or via or separate preparations), or sequentially or separately via separate pharmaceutical preparations.

Thus, according to the present invention, there is provided a pharmaceutical product comprising, in combination,

(a) a first active ingredient which is a compound of general formula

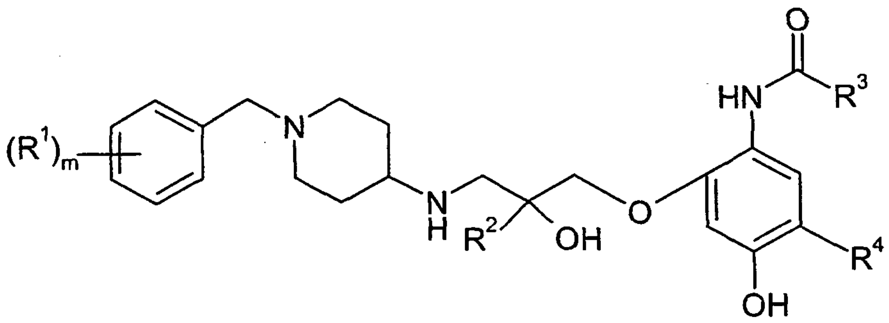

(I)

wherein

m is 0, 1 or 2;
each $R^1$ independently represents halogen;
$R^2$ represents a hydrogen atom or methyl;
$R^3$ represents the group $C_1$-$C_4$ alkyl;
and
$R^4$ represents hydrogen or halogen;

or a pharmaceutically acceptable salt thereof; and
(b) a second active ingredient which is a glucocorticosteroid.

The pharmaceutical product of the present invention may, for example, be a pharmaceutical composition comprising the first and second active ingredients in admixture. Alternatively, the pharmaceutical product may, for example, be a kit comprising a preparation of the first active ingredient and a preparation of the second active ingredient and, optionally, instructions for the simultaneous, sequential or separate administration of the preparations to a patient in need thereof.

In the context of the present specification, an alkyl substituent group or an alkyl moiety in a substituent group may be linear or branched.

The integer m is preferably 1 or 2.

Each $R^1$ independently represents halogen (e.g. chlorine, fluorine, bromine or iodine).

In one embodiment of the invention, m is 1 and $R^1$ represents a halogen atom, particularly a chlorine atom.

In a further embodiment, m is 1 and $R^1$ represents a halogen atom (e.g. chlorine) in the 4-position of the benzene ring relative to the carbon atom to which the $CH_2$ linking group is attached.

$R^2$ represents a hydrogen atom or methyl. In one embodiment of the present invention, $R^2$ represents methyl.

$R^3$ represents the group $C_1$-$C_4$ alkyl (e.g. methyl, ethyl, n-propyl, isopropyl, n-butyl, isobutyl, tert-butyl). Typically, $R^3$ is methyl or ethyl, particularly methyl.

$R^4$ represents hydrogen or halogen (e.g. fluorine, chlorine, bromine or iodine). In an embodiment of the present invention, $R^4$ represents hydrogen or chlorine.

The compound of formula (1) are capable of existing in stereoisomeric forms. It will be understood that the invention encompasses the use of all geometric and optical isomers of the compounds of formula (I) and mixtures thereof including racemates. The use of tautomers and mixtures thereof also form an aspect of the present invention. Preferred optical isomers are the (S)-enantiomers (i.e. compounds with the S configuration at the stereocentre with $R^2$ and OH attached).

The compounds of formula (I) according to the present invention may be synthesised using the procedures set out in WO01/98273 and WO03/051839.

The compounds of formulas (I) may be used in the form of a pharmaceutically acceptable salt thereof, preferably an acid addition salt such as a hydrochloride, hydrobromide, phosphate, sulfphate, acetate, ascorbate, benzoate, fumarate, hemifumarate, furoate, succinate, maleate, tartrate, citrate, oxalate, xinafoate, methanesulphonate or p-toluenesulphonate. A pharmaceutically acceptable salt also includes internal salt (zwitterionic) forms. Any reference to compounds of formula (I) or salts thereof also encompasses solvates of such compounds and solvates of such salts (e.g. hydrates).

It will be appreciated that the compounds of formula (I) and salts thereof may exist as zwitterions. Thus, whilst the compounds are drawn and referred to in the hydroxyl form, they may exist also in internal salt (zwitterionic) form. The representation of formula (I) and the examples of the present invention covers both hydroxyl and zwitterionic forms and mixtures thereof in all proportions.

In another embodiment of the present invention, the compound of formula (I) is selected from

*N*-{2-[((2*S*)-3-{[1-(4-chlorobenzyl)piperidin-4-yl]amino}-2-hydroxy-2-methylpropyl)oxy]-4-hydroxyphenyl} acetamide,

*N*-{5-Chloro-2-[((2*S*)-3-{[1-(4-chlorobenzyl) piperidin-4-yl] amino}-2-hydroxy-2-methylpropyl) oxy]-4-hydroxyphenyl} acetamide,

*N*-{5-Chloro-2-[((2*S*)-3-{[1-(4-chlorobenzyl)piperidin-4-yl]amino}-2-hydroxypropyl)oxy]-4-hydroxyphenyl} acetamide,

*N*-{5-Chloro-2-[((2*S*)-3-{[1-(4-chlorobenzyl)piperidin-4-yl]amino}-2-hydroxypropyl)oxy]-4-hydroxyphenyl} propaneamide, or

*N*-{5-Chloro-2-[((2*S*)-3-{[1-(4-chlorobenzyl)piperidin-4-yl]amino}-2-hydroxy-2-methylpropyl)oxy]-4-hydroxyphenyl} propaneamide,

or a pharmaceutically acceptable salt thereof.

In another embodiment of the present invention, the compound of formula (I) is a salt of *N*-{2-[((2*S*)-3-{[1-(4-chlorobenzyl)piperidin-4-yl]amino}-2-hydroxy-2-methylpropyl)oxy]-4-hydroxyphenyl} acetamide, for example hydrochloride, hydrobromide, phosphate, sulfphate, acetate, ascorbate, benzoate, fumarate, hemifumarate, furoate, succinate, maleate, tartrate, citrate, oxalate, xinafoate, methanesulphonate orp-toluenesulphonate salt. Salts with particularly good properties (e.g. favourable crystallinity and other physio properties suitable for e.g. being formulated in a dry powder formulation for pulmonary administration) are the benzoate, fumarate, or hemifumarate salts of *N*-{2-[((2*S*)-3-{[1-(4-chlorobenzyl)piperidin-4-yl]amino}-2-hydroxy-2-methylpropyl)oxy]-4-hydroxyphenyl} acetamide, including any forms of the salts referred to in the Examples.

In an embodiment of the invention, the compound of formula (I) or salt thereof has crystalline properties and is e.g. at least 50% crystalline, at least 60% crystalline, at least 70% crystalline or at least 80% crystalline. Crystallinity can be estimated by conventional X-ray diffractometry techniques.

In another embodiment of the invention, the compound of formula (I) or salt thereof is from 50%, 60%, 70%, 80% or 90% to 95%, 96%, 97%, 98%, 99% or 100% crystalline.

It should be noted that where X-ray powder diffraction peaks are expressed herein (in degrees 20), the margin of error is consistent with the United States Pharmacopeia general chapter on X-ray diffraction (USP941)- see the United States Pharmacopeia Convention. X-Ray Diffraction, General Test <941>. United States Pharmacopeial, 25th ed. Rockville, MD: United States Pharmacopeial Convention; 2002:2088-2089).

In an embodiment of the invention, the compound of formula (I) is a hemifumarate salt of *N*-{2-[((2*S*)-3-{[1-(-chlorobenzyl)piperidin-4-yl]amino}-2-hydroxy-2-methylpropyl)oxy]-4-hydroxyphenyl} acetamide which exhibits at least the following characteristic X-ray powder diffraction peaks (expressed in degrees 2θ):

(1) 6.2,10.7 and 12.5, or
(2) 6.2, 10.7 and 18.8, or
(3) 6.2, 10.7 and 18.0, or
(4) 6.2, 10.7, 12.5, 18.0 and 18.8, or
(5) 6.2, 10.7, 12.5, 18.0, 18.8, 19.7 and 19.8.

In another embodiment of the invention, the compound of formula (I) is a furoate salt of *N*-{2-[((2*S*)-3-{[1-(4-chlorobenzyl)piperidin-4-yl]amino}-2-hydroxy-2-methylpropyl)oxy]-4-hydroxyphenyl} acetamide which exhibits at least the following characteristic X-ray powder diffraction peaks (expressed in degrees 2θ):

(1) 6.3, 11.0 and 12.7, or
(2) 6.3, 10.7 and 12.7, or
(3) 6.3, 11.0, 12.7 and 15.9, or
(4) 6.3, 10.7, 11.0, 12.7, 13.9, 14.2 and 15.9, or
(5) 6.3, 10.7, 11.0, 12.7, 15.9, 17.7, 19.1, 19.7 and 25.5, or

(6) 6.3, 10.7, 11.0, 12.7, 13.9, 14.2, 15.9, 17.7, 19.1, 19.7, 19.9, 21.6 and 25.5.

In another embodiment of the invention, the compound of formula (I) is a furoate salt of *N*-{2-[((2*S*)-3-{[1-(4-chlorobenzyl)piperidin-4-yl]amino}-2-hydroxy-2-methylpropyl)oxy]-4-hydroxyphenyl}acetamide which exhibits at least the following characteristic X-ray powder diffraction peaks (expressed in degrees 2θ):

(1) 6.7, 11.0 and 13.4, or
(2) 6.7, 10.4, 11.0 and 13.4, or
(3) 6.7, 10.4, 12.4, 13.4 and 13.7, or
(4) 6.7, 10.4, 13.4 and 20.9, or
(5) 6.7, 10.4, 11.0, 12.4, 13.4, 13.7, 15.6, 16.0 and 17.6, or
(6) 6.7, 10.4, 11.0, 12.4, 13.4, 13.7, 15.6, 16.0, 16.1, 17.6, 18.0, 18.6, 18.9, 20.1, 20.9 and 23.4.

In another embodiment of the invention, the compound of formula (I) is a benzoate salt of *N*-{2-[((2*S*)-3-{[1-(4-chlorobenzyl)piperidin-4-yl]amino}-2-hydroxy-2-methylpropyl)oxy]-4-hydroxyphenyl} acetamide which exhibits at least the following characteristic X-ray powder diffraction peaks (expressed in degrees 2θ):

(1) 6.1, 10.7 and 19.3, or
(2) 6.1, 12.2 and 14.1, or
(3) 6.1, 10.7, 12.2, 14.1, 18.1 and 19.3, or
(4) 6.1, 10.7, 12.2, 14.1, 15.7, 18.1 and 19.3, or
(5) 6.1, 10.7, 12.2, 14.1, 15.1 and 19.3, or
(6) 6.1, 10.7, 12.2, 14.1, 15.1, 15.7, 18.1 and 19.3, or
(7) 6.1, 10.7, 12.2, 14.1, 15.1, 15.7, 18.1, 19.3, 21.2 and 24.6.

In another embodiment of the invention, the compound of formula (I) is a benzoate salt of *N*-{2-[((2*S*)-3-{[1-(4-chlorobenzyl)piperidin-4-yl]amino}-2-hydroxy-2-methylpropyl)oxy]-4-hydroxyphenyl} acetamide which exhibits at least the following characteristic X-ray powder diffraction peaks (expressed in degrees 2θ):

(1) 6.5, 9.3 and 10.5, or
(2) 6.5, 9.3, 17.6 and 17.8, or
(3) 6.5, 9.3, 10.5,12.0 and 12.4, or
(4) 6.5, 9.3, 10.5, 12.0, 12.4, 13.0, 13.6, 15.5, 17.6 and 17.8, or
(5) 6.5, 13.0 and 20.2, or
(6) 6.5, 9.3,10.5,12.0,12.4,13.0,13.6,15.5,17.6,17.8 and 19.2, or
(7) 6.5, 9.3, 10.5, 12.0, 12.4, 13.0, 13.6, 15.5, 17.6, 17.8, 19.2, 20.2, 22.8 and 26.0, or
(8) 6.5, 9.3, 10.5, 12.0, 12.4, 13.0, 13.6, 15.5, 17.6, 17.8, 19.2, 20.2, 22.8, 24.2, 26.0 and 30.7.

In a further embodiment of the invention, the compound of formula (I) is the furoate or benzoate salt of *N*-{5-Chloro-2-[((2*S*)-3-{[1-(4-chlorobenzyl)piperidin-4-yl]amino}-2-hydroxy-2-methylpropyl)oxy]-4-hydroxyphenyl} acetamide.

The second active ingredient in the combination of the present invention is a glucocorticosteroid. The glucocorticosteroid of the present invention may be any synthetic or naturally occurring glucocorticosteroid. Examples of glucocorticosetroid that may be used in accordance with the present invention include budesonide, fluticasone (e.g. as propionate ester), mometasone (e.g. as furoate ester), beclomethasone (e.g. as 17-propionate or 17,21-dipropionate esters), ciclesonide, loteprednol (as e.g. etabonate), etiprednol (e.g. as dicloacetate), triamcinolone (e.g. as acetonide), flunisolide, zoticasone, flumoxonide, rofleponide, butixocort (e.g. as propionate ester), prednisolone, prednisone, tipredane, steroid esters according to WO 2002/12265, WO 2002/12266 and WO 2002/88167 e.g. 6α,9α-difluoro-17α-[(2-furanylcarbonyl)oxy]-11β-hydroxy-16α-methyl-3-oxo-androsta-1,4-diene-17β-carbothioic acid S-fluoromethyl ester, 6α,9α-difluoro-11β-hydroxy-16α-methyl-3-oxo-17α-propionyloxy-androsta-1,4-diene-17β-carbothioic acid S-(2-oxo-tetrahydro-furan-3S-yl) ester and 6α,9α-difluoro-11β-hydroxy-16α-methyl-17α-[(4-methyl-1,3-thiazole-5-carbonyl)oxy]-3-oxo-androsta-1,4-diene-17β-carbothioic acid S-fluoromethyl ester, steroid esters such as those described in DE 4129535, steroids e.g. GSK870086, GSK685698, GSK799943 and those according to WO 2002/00679, WO 2005/041980.

In the context of the present specification, unless otherwise indicated any reference to a glucocorticosteroid includes all active salts, solvates or derivatives that may be formed from said glucocorticosteroid. Examples of possible salts or derivatives of glucocorticosteroids include; sodium salts, sulphobenzoates, phosphates, isonicotinates, acetates, propionates; dihydrogen phosphates, palmitates, pivalates, fumarates and pharmaceutically acceptable esters (e.g. $C_1$-$C_6$ alkyl esters). Glucocorticosteroids and active salts or derivatives thereof may also be in the form of their solvates,

e.g. hydrates.

In one embodiment of the present invention the glucocorticosteroid is budesonide. The chemical name for budesonide is 16,17-[butylidenebis(oxy)]-11,21-dihydroxy-pregna-1,4-diene-3,20-dione). Budesonide and its preparation is described, for example, in Arzneimittel-Forschung (1979), 29 (11), 1687-1690, DE 2,323,215 and US 3,929,768. Presently available formulations of budesonide are marketed under the tradename 'Entocort'.

The use of compounds of formula (I) are considered to demonstrate particularly surprising effects when used in combination with glucocorticosteroids, and in particular in combination with budesonide. For example, *in vivo* animal experiments indicate that a combination of a glucocorticosteriod and a compound of formula (I), at dose levels where either component alone does not significantly affect lung inflammation, in combination give significant reduction of inflammatory cell influx. The reduction in cell influx for the combination was greater than that expected from the additive effect of the two ingredients. This synergistic effect observed when combining the ingredients could be used, for example, to lower the therapeutic dose of steroid, or at the same dose, achieve enhanced efficacy on inflammation in comparison to the use of the steroid alone. The synergistic effect can be particularly advantageous where lower doses of steroid are desirable, for example in individuals that have acquired resistance to such steroids.

The pharmaceutical product of the present invention may further optionally comprise, as a third active ingredient, a bronchodilator. A bronchodilator is a drug that relaxes and dilates the bronchial passageways and improves the passage of air into the lungs. Examples of a bronchodilator which can be used in the present invention include a $\beta_2$- agonist or an anticholinergic compound.

$\beta_2$-agonists (also known as beta2 ($\beta_2$) adrenoreceptor agonists) may be used to alleviate symptoms of respiratory diseases by relaxing the bronchial smooth muscles, reducing airway obstruction, reducing lung hyperinflation and decreasing shortness of breath. The $\beta_2$-agonist of the present invention may be any compound or substance capable of stimulating the $\beta_2$-receptor and acting as a bronchodilator. Examples of $\beta_2$-agonists that may be used in the present invention include bambuterol, bitolterol, carbuterol, indacaterol, clenbuterol, fenoterol, formoterol, hexoprenaline, ibuterol, pirbuterol, procaterol, reproterol, salmeterol, sulphonterol, terbutaline, tolubuterol, TA 2005 (chemically identified as 2 (1H)-Quinolone, 8-hydroxy-5-[1-hydroxy-2-[[2-(4-methoxy-phenyl)-1-methylethyl]-amino]ethyl]-monohydrochloride, [R-(R*,R*)] also identified by Chemical Abstract Service Registry Number 137888-11-0 and disclosed in U.S. Patent No 4.579.854 (= CHF-4226), GSK159797, formanilide derivatives e.g. 3-(4-{[6-({(2R)-2-[3-(formylamino)-4-hydroxyphenyl]-2-hydroxyethyl}amino)hexyl]oxy}-butyl)-benzenesulfonamide as disclosed in WO 2002/76933, benzenesulfonamide derivatives e.g. 3-(4-{[6-({(2R)-2-hydroxy-2-[4-hydroxy-3-(hydroxy-methyl)phenyl]ethyl}amino)-hexyl]oxy}butyl)benzenesulfonamide as disclosed in WO 2002/88167, aryl aniline receptor agonists such as disclosed in WO 2003/042164 and WO 2005/025555, and indole derivatives such as disclosed in WO 2004/032921.

In one aspect, the $\beta_2$ -agonist of the invention is a long acting $\beta_2$ -agonist, i.e. a $\beta_2$ -agonist with activity that persists for more than 12 hours. Examples of long acting $\beta_2$ -agonists include formoterol, bambuterol and salmeterol.

In the context of the present specification, unless otherwise stated, any reference to a bronchodilator (including $\beta_2$-agonists, long acting $\beta_2$-agonists and anticholinergic compounds) includes active salts, solvates or derivatives that may be formed from said bronchodilator and any enantiomers and mixtures thereof, including racemates. Examples of possible salts or derivatives are acid addition salts such as the salts of hydrochloric acid, hydrobromic acid, sulphuric acid, phosphoric acid, methanesulphonic acid, acetic acid, fumaric acid, succinic acid, lactic acid, citric acid, tartaric acid, 1-hydroxy-2-naphthalenecarboxylic acid, maleic acid, and pharmaceutically acceptable esters (e.g. $C_1$-$C_6$ alkyl esters). The bronchodilator (including salts and derivatives thereof) may also be in the form of a solvate, e.g. a hydrate.

Examples of an anticholinergic compound include ipratropium (e.g. as bromide), tiotropium (e.g. as bromide), oxitropium (e.g. as bromide), tolterodine, AD-237 (Arakis), and quinuclidine derivatives as disclosed in US 2003/0055080.

In an embodiment of the present invention, the bronchodilator is formoterol. The chemical name for formoterol is *N*-[2-hydroxy-5-[(1)-1-hydroxy-2-[[(1)-2-(4-methoxyphenyl)-1-methylethyl]amino]ethyl]phenyl]-formamide. The preparation of formoterol is described, for example, in WO 92/05147. As will be clear from the above, the term formoterol is intended to include all pharmaceutically acceptable salts thereof. In one aspect of this embodiment, the bronchodilator is formoterol fumarate, for example formoterol fumarate dihydrate.

As emphasised above, it will be understood that the invention encompasses the use of all optical isomers of formoterol and mixtures thereof including racemates. Thus for example, the term formoterol encompasses *N*-[2-hydroxy-5-[(1*R*)-1-hydroxy-2-[[(1*R*)-2-(4-methoxyphenyl)-1-methylethyl]amino]ethyl]phenyl]-formamide, *N*-[2-hydroxy-5-[(1*S*)-1-hydroxy-2-[[(1*S*)-2-(4-methoxyphenyl)-1-methylethyl]amino]ethyl]phenyl]-formamide or a mixture of such enantiomers, including a racemate.

In a further embodiment of the present invention, the bronchodilator is indacaterol. As will be clear from the above, the term indacaterol is intended to include all pharmaceutically acceptable salts thereof, including for example, indacaterol maleate and indacaterol hydrochloride.

The compound of formula (I) or a pharmaceutically acceptable salt thereof (first active ingredient), glucocorticosteroid (second active ingredient) and optionally bronchodilator (third active ingredient) of the present invention may be administered simultaneously, sequentially or separately to treat respiratory diseases. By sequential it is meant that

the active ingredients are administered, in any order, one immediately after the other. They still have the desired effect if they are administered separately, but when administered in this manner they are generally administerted less than 4 hours apart, more conveniently less than two hours apart, more conveniently less than 30 minutes apart and most conveniently less than 10 minutes apart.

The active ingredients of the present invention may be administered by oral or parenteral (e.g. intravenous, subcutaneous, intramuscular or intraarticular) administration using conventional systemic dosage forms, such as tablets, capsules, pills, powders, aqueous or oily solutions or suspensions, emulsions and sterile injectable aqueous or oily solutions or suspensions. The active ingredients may also be administered topically (to the lung and/or airways) in the form of solutions, suspensions, aerosols and dry powder formulations. These dosage forms will usually include one or more pharmaceutically acceptable ingredients which may be selected, for example, from adjuvants, carriers, binders, lubricants, diluents, stabilising agents, buffering agents, emulsifying agents, viscosity-regulating agents, surfactants, preservatives, flavourings and colorants. As will be understood by those skilled in the art, the most appropriate method of administering the active ingredients is dependent on a number of factors.

In one embodiment of the present invention the active ingredients are administered via separate pharmaceutical preparations.

Therefore, in one aspect, the present invention provides a kit comprising a preparation of a first active ingredient which is a compound of formula (I) or a pharmaceutically acceptable salt thereof, and a preparation of a second active ingredient which is a glucocorticosteroid, and optionally instructions for the simultaneous, sequential or separate administration of the preparations to a patient in need thereof. The kit may further optionally comprise a preparation of a third active ingredient, which is a bronchodilator.

In another embodiment the active ingredients may be administered via a single pharmaceutical composition. Therefore, the present invention further provides a pharmaceutical composition comprising, in admixture, a first active ingredient which is compound of formula (I) or pharmaceutically acceptable salt thereof, and a second active ingredient which is a glucocorticosteroid. The pharmaceutical composition may further optionally comprise a third active ingredient, which is a bronchodilator. The present invention also provides a process for the preparation of a pharmaceutical composition which comprises mixing the first active ingredient with the second active ingredient and optionally with the third active ingredient.

The pharmaceutical compositions of the present invention may be prepared by mixing the first active ingredient and the second active ingredient with a pharmaceutically acceptable adjuvant, diluent or carrier, and, optionally, the third active ingredient. Therefore, in a further aspect of the present invention there is provided a process for the preparation of a pharmaceutical composition, which comprises mixing a compound of formula (I) or pharmaceutically acceptable salt thereof, with a glucocorticosteroid, and a pharmaceutically acceptable adjuvant, diluent or carrier, and, optionally, a bronchodilator.

It will be understood that the therapeutic dose of each active ingredient administered in accordance with the present invention will vary depending upon the particular active ingredient employed, the mode by which the active ingredient is to be administered, and the condition or disorder to be treated.

In one embodiment of the present invention, the first, second (and when present, the third) active ingredients of the present invention are each administered by inhalation. In this aspect, the active ingredients are inhaled simultaneously, sequentially or separately.

Throughout the specification, the amount of the active ingredients used relate to unit doses unless explicitly defined differently.

When administered via inhalation the dose of the first active ingredient (compound of formula (I) or a pharmaceutically acceptable salt thereof), will generally be in the range of from 0.1 μg to 10000 μg, 0.1 to 5000 μg, 0.1 to 1000 μg, 0.1 to 500 μg, 0.1 to 200 μg, 0.1 to 200 μg, 0.1 to 100 μg, 0.1 to 50 μg, 5 μg to 5000 μg, 5 to 1000 μg, 5 to 500 μg, 5 to 200 μg, 5 to 100 μg, 5 to 50 μg, 10 to 5000 μg, 10 to 1000 μg, 10 to 500 μg, 10 to 200 μg, 10 to 100 μg, 10 to 50 μg, 20 to 5000 μg, 20 to 1000 μg, 20 to 500 μg, 20 to 200 μg, 20 to 100 μg, 20 to 50 μg, 50 to 5000 μg, 50 to 1000 μg, 50 to 500 μg, 50 to 200 μg, 50 to 100 μg, 100 to 5000 μg, 100 to 1000 μg or 100 to 500 μg.

The first active ingredient (compound of formula (I) or a pharmaceutically acceptable salt thereof) may also be administered orally. Oral administration of the CCR1 receptor antagonist may for example be used in a pharmaceutical product or kit wherein the other active ingredient(s) are administered by inhalation.

When administered via inhalation the dose of the second active ingredient (glucocorticosteroid), will generally be in the range of from 0.1 microgram (μg) to 1000 μg, 0.1 to 500 μg, 0.1 to 200 μg, 0.1 to 100 μg, 0.1 to 50 μg, 0.1 to 5 μg, 5 to 1000 μg, 5 to 500 μg, 5 to 200 μg, 5 to 50 μg, 5 to 10 μg, 10 to 1000 μg, 10 to 500 μg, 10 to 200 μg, 10 to 100 μg, 10 to 50 μg, 20 to 1000 μg, 20 to 500 μg, 20 to 200 μg, 20 to 100 μg, 20 to 50 μg, 50 to 1000 μg, 50 to 500 μg, 50 to 200 μg, 50 to 100 μg, 100 to 1000 μg, or 100 to 500 μg.

In one embodiment, the amount of the first active agent used is in the range 1 μg to 200 μg, and that of the second agent in the range 1 μg to 200 μg.

The molar ratio of the second active ingredient to the first active ingredient in a dose may typically be in the

range of 1:10 to 10:1. Preferably the ratio is in the range 1:1 to 10:1, and preferably still, in the range 5:1 to 20:1.

When present, the third active ingredient (bronchodilator), may conveniently be administered by inhalation at a dose generally in the range of from 0.1 to 100 μg, 0.1 to 50 μg, 0.1 to 40 μg, 0.1 to 30 μg, 0.1 to 20 μg, 0.1 to 10 μg, 5 to 100 μg, 5 to 50 μg, 5 to 40 μg, 5 to 30 μg, 5 to 20 μg, 5 to 10 μg, 10 to 100 μg, 10 to 50 μg, 10 to 40 μg, 10 to 30 μg, or 10 to 20 μg. In an embodiment of the present invention, the dose of the third active ingredient is in the range 1 to 30 μg.

The doses of the first and second (and where present the third) active ingredients will generally be administered from 1 to 4 times a day, conveniently once or twice a day, and most conveniently once a day.

In one embodiment, the present invention provides a pharmaceutical product comprising, in combination, a first active ingredient which is a compound of formula (I) or a pharmaceutically acceptable salt thereof, and a second active ingredient which is a glucocorticosteroid, and optionally a third active ingredient which is bronchodilator, wherein each active ingredient is formulated for inhaled administration.

The active ingredients are conveniently administered via inhalation (e.g. topically to the lung and/or airways) in the form of solutions, suspensions, aerosols or dry powder formulations. Administration may be by inhalation orally or intranasally. The active ingredients are preferably adapted to be administered, either together or individually, from a dry powder inhaler, pressurised metered dose inhaler, or a nebuliser.

The active ingredients may be used in admixture with one or more pharmaceutically acceptable additives, diluents or carriers. Examples of suitable diluents or carriers include lactose (e.g. the monohydrate), dextran, mannitrol or glucose.

Metered dose inhaler devices may be used to administer the active ingredients, dispersed in a suitable propellant and with or without additional excipients such as ethanol, a surfactants, a lubricant, an anti-oxidant or a stabilising agent. Suitable propellants include hydrocarbon, chlorofluorocarbon and hydrofluoroalkane (e.g. heptafluoroalkane) propellants, or mixtures of any such propellants. Preferred propellants are P134a and P227, each of which may be used alone or in combination with other propellants and/or surfactant and/or other excipients. Nebulised aqueous suspensions or, preferably, solutions may also be employed, with or without a suitable pH and/or tonicity adjustment, either as a unit-dose or multi-dose formulations.

Dry powder inhalers may be used to administer the active ingredients, alone or in combination with a pharmaceutically acceptable carrier, in the later case either as a finely divided powder or as an ordered mixture. The dry powder inhaler may be single dose or multi-dose and may utilise a dry powder or a powder-containing capsule.

When the active ingredients are adapted to be administered, either together or individually, via a nebuliser they may be in the form of a nebulised aqueous suspension or solution, with or without a suitable pH or tonicity adjustment, either as a single dose or multidose device.

Metered dose inhaler, nebuliser and dry powder inhaler devices are well known and a variety of such devices are available.

In an embodiment of the present invention, the compound of formula (I) or pharmaceutically salt thereof may be administered orally and the other active ingredient(s) administered by inhalation.

The present invention further provides a pharmaceutical product, kit or pharmaceutical composition according to the invention for simultaneous, sequential or separate use in therapy.

The present invention further provides the use of a pharmaceutical product, kit or pharmaceutical composition according to the invention in the manufacture of a medicament for the treatment of a respiratory disease, in particular chronic obstructive pulmonary disease or asthma.

The present invention still further provides a method of treating a respiratory disease which comprises simultaneously, sequentially or separately administering:

(a) a (therapeutically effective) dose of a first active ingredient which is a compound of formula (I) or a pharmaceutically acceptable salt thereof; and
(b) a (therapeutically effective) dose of a second active ingredient which is a glucoccorticosteroid; and optionally
(c) a (therapeutically effective) dose of a third active ingredient which is a bronchodilator; to a patient in need thereof.

In the context of the present specification, the term "therapy" also includes "prophylaxis" unless there are specific indications to the contrary. The terms "therapeutic" and "therapeutically" should be construed accordingly. Prophylaxis is expected to be particularly relevant to the treatment of persons who have suffered a previous episode of, or are otherwise considered to be at increased risk of, the condition or disorder in question. Persons at risk of developing a particular condition or disorder generally include those having a family history of the condition or disorder, or those who have been identified by genetic testing or screening to be particularly susceptible to developing the condition or disorder.

The present invention will now be further understood by reference to the following illustrative examples, wherein

FIG 1 shows the results of a cell influx experiment in LPS-challenged rats using a combination of the present invention.

**General methods**

$^1$H NMR spectra were recorded on a Varian Unity Inova 400 or a Varian Mercury VX 300 and data are quoted in the form of delta values, given in parts per million (ppm) relative to tetramethylsilane (TMS) as an internal standard.
The central solvent peak of chloroform-*d* ($\delta_H$ 7.27 ppm), acetone-$d_6$ ($\delta_H$ 2.05 ppm), or DMSO-$d_6$ ($\delta_H$ 2.50 ppm) were used as internal standard.
Low resolution mass spectra and accurate mass determination were recorded on an Agilent MSD 1100 LC-MS system equipped with APCI /ESI ionisation chambers.
All solvents and commercial reagents were laboratory grade and used as received.
The following abbreviations are used:

| | |
|---|---|
| DMSO | dimethyl sulfoxide; |
| DMF | *N*-dimethylformamide; |
| THF | tetrahydrofuran; |
| TFA | trifluoroacetic acid; |

X-Ray Powder Diffraction Analyses

X-ray powder diffraction (XRPD) analyses may be performed on samples prepared according to standard methods (see for example Giacovazzo et al., eds., Fundamentals of Crystallography, Oxford University Press (1992); Jenkins & Snyder, eds., Introduction to X-Ray Powder Diffractometry, John Wiley & Sons, New York (1996); Bunn, ed., Chemical Crystallography, Clarendon Press, London (1948); and Klug & Alexander eds., X-ray Diffraction Procedures, John Wiley & Sons, New York (1974)).
An X-ray powder diffraction pattern of the hemi-fumarate salt described in Example 1 above (in anhydrous form) was obtained as described below:
A Bragg-Brentano parafocusing powder X-ray diffractometer using monochromatic CuKa radiation (45 kV and 40 mA) was used for the analyses. The primary optics contained soller slits and an automatic divergence slit. Flat samples were prepared on zero background plates that were rotated during the meausurements. The secondary optics contained soller slits, an automatic anti scatter slit, a receiving slit and a monochromator. The diffracted signal was detected with a proportional xenon-filled detector. Diffraction patterns were collected between 2° ≤ 2θ (theta) ≤ 40° in a continous scan mode with a step size of 0.016° 2θ at a rate of 4° 2θ per minute. Raw data were stored electronically. Evaluation was performed on raw or smoothed diffraction patterns.
A Panalytical X'pert PRO MPD θ-θ diffractometer in reflection mode was used for the above-mentioned measurements. A person skilled in the art can set up instrumental parameters for a powder X-ray diffractometer so that diffraction data comparable to the data presented can be collected.

**Prepration of MIP-1α chemokine receptor antagonists**

**Example 1(a)**

***N*-{2-[((2*S*)-3-{[1-(4-chlorobenzyl)piperidin-4-yl]amino}-2-hydroxy-2-methylpropyl)oxy]-4-hydroxyphenyl} acetamide hemi-fumarate (2:1 salt)**

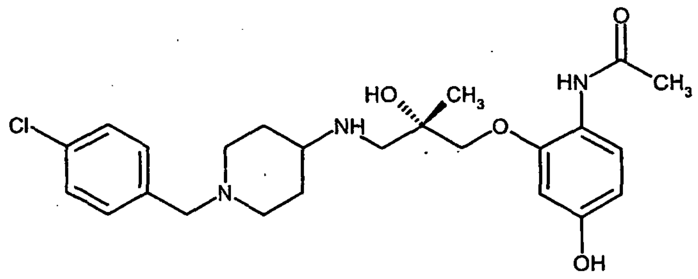

To a stirred solution of crude *N*-{2-[((2*S*)-3-{[1-(4-chlorobenzyl)piperidin-4-yl]amino}-2-hydroxy-2-methylpropyl) oxy]-4-hydroxyphenyl}acetamide (24.0 g, 36.5 mmol; obtained by extraction at pH 9 from the corresponding salt with trifluoroacetic acid as described in Example 1 of WO 03/051839) in methanol (240 ml), a solution of fumaric acid (2.13 g, 18.3 mmol) in methanol (55 ml) was added over a period of 15 minutes. It was observed that a precipitate began to form when about two thirds of the fumaric acid solution had been added. When all the fumaric acid solution had been added, the stirring was stopped and the reaction mixture was left overnight at ambient temperature (20°C) in a closed flask. The precipitate was isolated on a filter funnel, washed with methanol (3 x 50 ml) and dried *in vacuo* at 60°C overnight to give the titled salt as an off-white solid (14.0 g, 73%).
$^1$H NMR (399.99 MHz, dmso) δ 8.91 (s, 1H), 7.48 (d, *J* = 8.6 Hz, 1H), 7.38 (d, *J* = 8.5 Hz, 2H), 7.31 (d, *J* = 8.4 Hz, 2H), 6.50 (s, 1H), 6.42 (d, *J* = 2.5 Hz, 1H), 6.31 (dd, *J* = 8.6, 2.5 Hz, 1H), 3.79 (s, *strongly coupled AB-system,* 2H), 3.44 (s, 2H), 2.88 (d, *J* = 12.2 Hz, 1H), 2.82-2.72 (m, 3H), 2.64 - 2.55 (m, 1H), 2.02 (s, 3H), 2.00 -1.92 (m, 2H), 1.91 - 1.83 (m, 2H), 1.47 -1.35 (m, 2H), 1.23 (s, 3H)
APCI-MS: m/z 462 [MH+]
The stoichiometry, base to acid, of 2:1 was confirmed by NMR.
The hemi-fumarate salt exhibits at least the following characteristic X-ray powder diffraction (XRPD) peaks (expressed in degrees 2θ) (the margin of error being consistent with the United States Pharmacopeia general chapter on X-ray diffraction (USP941) - see the United States Pharmacopeia Convention. X-Ray Diffraction, General Test <941>. United States Pharmacopeia, 25th ed. Rockville, MD: United States Pharmacopeial Convention; 2002:2088-2089):

(1) 6.2,10.7 and 12.5, or
(2) 6.2, 10.7 and 18.8, or
(3) 6.2,10.7 and 18.0, or
(4) 6.2, 10.7, 12.5, 18.0 and 18.8, or
(5) 6.2, 10.7, 12.5, 18.0, 18.8, 19.7 and 19.8.

<u>**Example 1(b)**</u>

**Preparation of *N*-{2-[((2*S*)-3-{[1-(4-chlorobenzyl)piperidin-4-yl]amino}-2-hydroxy-2-methylpropyl)oxy]-4-hydroxyphenyl}acetamide benzoate (1:1 salt), Form A**

(a) Hot solutions of *N*-{2-[((2*S*)-3-{[1-(4-chlorobenzyl)piperidin-4-yl]amino}-2-hydroxy-2-methylpropyl)oxy]-4-hydroxyphenyl}acetamide (which may be prepared by processes described in WO 03/051839; 462 mg, 1.0 mmol) in ethyl acetate (10 ml) and benzoic acid (244 mg, 2.0 mmol) in ethyl acetate (10 ml) were mixed. The resulting mixture was left to cool down to ambient temperature (20°C) in a closed vial. A white precipitate was formed without turbidity. After standing at ambient temperature overnight the precipitate obtained was washed with ethyl acetate (3x10 ml) and dried *in vacuo* at 60°C overnight to give the titled salt as an off-white solid (506 mg, 86%). The salt contained traces of ethyl acetate.

$^1$H NMR (399.99 MHz, acetone-$d_6$) δ 8.77 (s, 1H), 8.07 - 8.04 (m, 2H), 7.83 (d, *J* = 8.7 Hz, 1H), 7.55 - 7.50 (m, 1H), 7.46 - 7.41 (m, 2H), 7.36 - 7.31 (m, 4H), 6.52 (d, *J* = 2.6 Hz, 1H), 6.40 (dd, *J* = 8.7, 2.6 Hz, 1H), 3.97 (d, *J* = 9.3 Hz, 1H), 3.89 (d, *J* = 9.3 Hz, 1H), 3.48 (s, 2H), 3.29 (d, *J* = 12.1 Hz, 1H), 2.94 (d, *J* = 12.2 Hz, 1H), 2.91 - 2.77 (m, 3H), 2.09 - 2.00 (m, 4H), 1.98 (s, 3H), 1.72 - 1.59 (m, 2H), 1.30 (s, 3H)
APCI-MS: m/z 462 [MH+]
The stoichiometry, base to acid, of 1:1 was confirmed by NMR.

Further quantities of the titled salt were prepared by the following method:

(b) Hot solutions of *N*-{2-[((2*S*)-3-{[1-(4-chlorobenzyl)piperidin-4-yl]amino}-2-hydroxy-2-methylpropyl)oxy]-4-hydroxyphenyl}acetamide (4.0 g, 8.65 mmol) in ethyl acetate (75 ml) and benzoic acid (1.16 g, 9.5 mmol) in ethyl acetate (75 ml) were mixed. When the resulting mixture had cooled down to ambient temperature (20°C) it was seeded with a particle of the titled salt obtained in (a) above and was left overnight in a closed flask. The precipitate obtained was washed with ethyl acetate (3 x 50 ml) and dried *in vacuo* at 60°C overnight to give the titled salt as an off-white solid (4.41 g, 87%). The salt contained traces of ethyl acetate.

The benzoate Form A salt exhibits at least the following characteristic X-ray powder diffraction (XRPD) peaks (expressed in degrees 2θ) (the margin of error being consistent with the United States Pharmacopeia general chapter on X-ray diffraction (USP941) - see the United States Pharmacopeia Convention. X-Ray Diffraction, General Test <941>. United States Pharmacopeia, 25th ed. Rockville, MD: United States Pharmacopeial Convention; 2002:2088-2089):

(1) 6.1, 10.7 and 19.3, or
(2)6.1,12.2 and 14.1, or
(3) 6.1, 10.7, 12.2, 14.1, 18.1 and 19.3, or
(4) 6.1, 10.7, 12.2, 14.1, 15.7, 18.1 and 19.3, or
(5) 6.1,10.7,12.2,14.1,15.1 and 19.3, or
(6) 6.1, 10.7, 12.2, 14.1, 15.1, 15.7, 18.1 and 19.3, or
(7) 6.1, 10.7, 12.2, 14.1, 15.1, 15.7, 18.1, 19.3, 21.2 and 24.6.

**Preparation of *N*-{2-[((2*S*)-3-{[1-(4-chlorobenzyl)piperidin-4-yl]amino}-2-hydroxy-2-methylpropyl)oxy]-4-hydroxyphenyl}acetamide benzoate (1:1 salt), Form B**

(a) The benzoate salt prepared by the method of Example 1(b) (Form A, 10 to 15 mg) was placed in a Differential Scanning Calorimetry pan (with a lid crimped) and using a heating rate of 5 K.min-1, heated until a temperature of 155°C was reached. Once the salt had melted (an onset melting temperature of 146.5°C was recorded under the conditions used), the melted sample was cooled down at a rate of 5 K.min-1 to ambient temperature (20°C). Then the same pan was heated again at a heating rate of 5 K.min-1 until a temperature of 151°C was reached and the scan recorded an isotherm at 148°C over a 10 minute period. The pan was then cooled rapidly to ambient temperature resulting in the formation of crystals which were subsequently confirmed by X-ray powder diffraction (XRPD) to be a new physical form of *N*-{2-[((2*S*)-3-{[1-(4-chlorobenzyl)piperidin-4-yl]amino}-2-hydroxy-2-methylpropyl)oxy]-4-hydroxyphenyl}acetamide benzoate (Form B). Some amorphous benzoate salt may be formed as a by-product of the process.

(b) The Form B salt described in (a) above was also prepared by dissolving, in a vial, 20%w of a sample of the benzoate salt prepared by the method of Example 1(b) (Form A) in a solvent such as methanol (>20 mg/ml), ethanol (>20 mg/ml), n-propanol (>20 mg/ml), isopropanol (8.5 mg/ml) or acetone (9.6 mg/ml). The figures in brackets indicate the estimated solubility of the salt in these solvents. The vial was then sealed and the suspension was homogenised at ambient temperature (20°C) using a magnet. Stirring and temperature were maintained for a period of at least 7 days after which time a sample of the material obtained was dried and tested by XRPD. XRPD confirmed that there had been complete transformation of Form A to Form B.

(c) The Form B salt described in (a) above was also prepared by dissolving benzoate salt prepared by the method of Example 1(6) (Form A) (22.0g, 37.7mmol) and benzoic acid (0.46 g, 3.8 mmol) in hot 2-propanol (190 ml) in a round-bottomed flask to give a reddish solution. The flask was rotated using a Rotavapor device on a waterbath at 40°C until the solution had cooled down to 40°C, whereupon it was seeded with some crystals of the Form B salt. The waterbath was allowed to cool down slowly to ambient temperature overnight while the flask was rotating and the mixture was seeded occasionally with some crystals of the Form B salt. A pink precipitate which formed was isolated by suction, washed with 2-propanol (2 x 50 ml) and dried *in vacuo* at 100°C for 20 hours to give the titled salt (as confirmed by XRPD) as a pale pink solid (18.5 g, 84%). The salt contained traces of 2-propanol.

$^1$H NMR (299.95 MHz, DMSO-$d_6$) δ 8.87 (s, 1H), 7.96 - 7.91 (m, 2H), 7.59 - 7.52 (m, 1H), 7.49 - 7.47 (m, 1H), 7.46 - 7.42 (m, 2H), 7.36 (d, *J* = 8.6 Hz, 2H), 7.29 (d, *J* = 8.6 Hz, 2H), 6.39 (d, *J* = 2.5 Hz, 1H), 6.29 (dd, *J* = 8.5, 2.5 Hz, 1H), 3.78 - 3.72 (m, 2H), 3.41 (s, 2H), 2.79 - 2.66 (m, 4H), 1.98 (s, 3H), 1.97 - 1.88 (m, 2H), 1.85 - 1.76 (m, 2H), 1.41 -

1.25 (m, 2H), 1.19 (s, 3H)

APCI-MS: m/z 462 [MH+]

The stoichiometry, base to acid, of 1:1 was confirmed by NMR.

The benzoate Form B salt exhibits at least the following characteristic X-ray powder diffraction (XRPD) peaks (expressed in degrees 2θ) (the margin of error being consistent with the United States Pharmacopeia general chapter on X-ray diffraction (USP941) - see the United States Pharmacopeia Convention. X-Ray Diffraction, General Test <941>. United States Pharmacopeia, 25th ed. Rockville, MD: United States Pharmacopeial Convention; 2002:2088-2089):

(1) 6.5, 9.3 and 10.5, or
(2) 6.5, 9.3, 17.6 and 17.8, or
(3) 6.5, 9.3, 10.5,12.0 and 12.4, or
(4) 6.5, 9.3, 10.5, 12.0, 12.4, 13.0, 13.6, 15.5, 17.6 and 17.8, or
(5) 6.5, 13.0 and 20.2, or
(6) 6.5, 9.3, 10.5, 12.0, 12.4, 13.0, 13.6, 15.5, 17.6,17.8 and 19.2, or
(7) 6.5, 9.3, 10.5, 12.0, 12.4,13.0, 13.6, 15.5, 17.6, 17.8, 19.2, 20.2, 22.8 and 26.0, or
(8) 6.5,9.3,10.5,12.0,12.4,13.0,13;6,15.5,17.6, 17.8, 19.2; 20.2, 22.8, 24.2, 26.0 and 30.7.

<u>**Example 1(c)**</u>

**Preparation of *N*-{2-[((2*S*)-3-{[1-(4-chlorobenzyl)piperidin-4-yl}amino}-2-hydroxy-2-methylpropyl)oxy]-4-hydroxyphenyl}acetamide furoate (1:1 salt), Form A**

(a) To a stirred solution of *N*-{2-[((2*S*)-3-{[1-(4-chlorobenzyl)piperidin-4-yl]amino}-2-hydroxy-2-methylpropyl)oxy]-4-hydroxyphenyl}acetamide (which may be prepared by processes described in WO 03/051839; 46 mg, 0.1 mmol) and furoic acid (23 mg, 0.2 mmol) in methanol (0.2 ml) contained in a vial, diethylether (5 ml) was added and the vial was closed. The resulting mixture was stirred for 3 days and a precipitate that formed was isolated, washed with diethylether and dried *in vacuo* to give an off-white solid (38 mg). The solid contained the titled salt as a crystalline material together with some amorphous salt. The titled salt contained trace amounts of diethylether.

$^1$H NMR (299.946 MHz, DMSO-$d_6$) δ 8.92 (s, 1H), 7.75 - 7.73 (m, 1H), 7.46 (d, *J* = 8.6 Hz, 1H), 7.37 (d, *J* = 4.4 Hz, 2H), 7.29 (d, *J* = 4.4 Hz, 2H), 6.97 - 6.94 (m, 1H), 6.54 (dd, *J* = 3 .4, 1.7 Hz, 1H), 6.40 (d, *J* = 2.4 Hz, 1H), 6.29 (dd, *J* = 8.6, 2.4 Hz, 1H), 3.78 (s, 2H), 3.43 (s, 2H), 2.93 (d, *J* = 12.1 Hz, 1H), 2.84 - 2.71 (m, 3H), 2.70 - 2.58 (m, 1H), 1.99 (s, 3H), 1.96 - 1.83 (m, 4H), 1.51-1.34 (m, 2H), 1.22 (s, 3H)

APCI-MS: m/z 462 [MH+]

The stoichiometry, base to acid, of 1:1 was confirmed by NMR.

Further quantities of the titled salt were prepared by the following method:

(b) To a solution of *N*-{2-[((2*S*)-3-{[1-(4-chlorobenzyl)piperidin-4-yl]amino}-2-hydroxy-2-methylpropyl)oxy]-4-hydroxyphenyl}acetamide (230 mg, 0.5 mmol) in methanol (0.5 ml) contained in a vial, furoic acid (62 mg, 0.55 mmol) was added as a solid. The mixture was shaken until a solution was obtained. The solution was diluted with ethyl acetate (6ml), seeded with a particle of the titled salt obtained in (a) above and was left overnight in the closed vial. The precipitate obtained was washed with ethyl acetate and dried *in vacuo* at 60°C overnight to give the titled salt as an off-white solid (200 mg, 70%). The titled salt contained trace amounts of ethyl acetate.

$^1$H NMR (299.946 MHz, DMSO-$d_6$) δ 8.94 (s, 1H), 7.73 - 7.71 (m, 1H), 7.47 (d, *J* = 8.6 Hz, 1H), 7.37 (d, *J* = 8.4 Hz, 2H), 7.30 (d, *J* = 8.4 Hz, 2H), 6.94 - 6.91 (m, 1H), 6.52 (dd, *J* = 3.3, 1.8 Hz, 1H), 6.40 (d, *J* = 2.2 Hz, 1H), 6.30 (dd, *J* = 8.6, 2.2 Hz, 1H), 3.78 (s, 2H), 3.43 (s, 2H), 2.97 (d, *J* = 11.9 Hz, 1H), 2.87 - 2.61 (m, 4H), 1.98 (s, 3H), 1.96 - 1.85 (m, 4H), 1.53 - 1.38 (m, 2H), 1.23 (s, 3H)

APCI-MS: m/z 462 [MH+]

The stoichiometry, base to acid, of 1:1 was confirmed by NMR.

The furoate Form A salt exhibits at least the following characteristic X-ray powder diffraction (XRPD) peaks (expressed in degrees 2θ) (the margin of error being consistent with the United States Pharmacopeia general chapter on X-ray diffraction (USP941) - see the United States Pharmacopeia Convention. X-Ray Diffraction, General Test <941>. United States Pharmacopeia, 25th ed. Rockville, MD: United States Pharmacopeial Convention; 2002:2088-2089):

(1) 6.3, 11.0 and 12.7, or

(2) 6.3, 10.7 and 12.7, or
(3) 6.3, 11.0, 12.7 and 15.9, or
(4) 6.3, 10.7, 11.0, 12.7, 13.9, 14.2 and 15.9, or
(5) 6.3, 10.7, 11.0, 12.7, 15.9, 17.7, 19.1, 19.7 and 25.5, or
(6) 6.3, 10.7, 11.0, 12.7, 13.9, 14.2, 15.9, 17.7, 19.1, 19.7, 19.9, 21.6 and 25.5.

**Preparation of *N*-{2-[((2*S*)-3-{[1-(4-chlorobenzyl)piperidin-4-yl]amino}-2-hydroxy-2-methylpropyl)oxy]-4-hydroxyphenyl}acetamide furoate (1:1 salt), Form B**

(a) Form B was prepared by dissolving, in a vial, 20%w of a sample of the furoate salt prepared by the method of Example 1(b) (Form A) in a solvent such as ethanol (16 mg/ml) or 2-butanol (8 mg/ml). The figures in brackets indicate the estimated solubility of the salt in these solvents. The vial was then sealed and the suspension was homogenised at ambient temperature (20°C) using a magnet. Stirring and temperature were maintained for a period of at least 7 days after which time a sample of the material obtained was dried and tested by XRPD. XRPD confirmed that there had been complete transformation of Form A to Form B.

Further quantities of the titled salt were prepared by the following method:

(b) Solutions of *N*-{2-[((2*S*)-3-{[1-(4-chlorobenzyl)piperidin-4-yl]amino}-2-hydroxy-2-methylpropyl)oxy]-4-hydroxyphenyl}acetamide (46 mg, 0.10 mmol) in 2-butanol (0.5 ml) and furoic acid (12.5 mg, 0.11 mmol) in 2-butanol (0.5 ml) were mixed and seeded with some crystals of Form B. The mixture was set aside in a closed vial at ambient temperature for 3 days. The precipitate obtained was washed with 2-butanol and dried *in vacuo* at 60°C overnight to give the titled salt as an off-white solid. The salt contained traces of 2-butanol.

The identity and stoichiometry, base to acid, of 1:1 were confirmed by NMR.
The furoate Form B salt exhibits at least the following characteristic X-ray powder diffraction (XRPD) peaks (expressed in degrees 2θ) (the margin of error being consistent with the United States Pharmacopeia general chapter on X-ray diffraction (USP941) - see the United States Pharmacopeia Convention. X-Ray Diffraction, General Test <941>. United States Pharmacopeia, 25th ed. Rockville, MD: United States Pharmacopeial Convention; 2002:2088-2089):

(1) 6.7, 11.0 and 13.4, or
(2) 6.7, 10.4, 11.0 and 13.4, or
(3) 6.7, 10.4, 12.4, 13.4 and 13.7, or
(4) 6.7,10.4, 13.4 and 20.9, or
(5) 6.7,10.4, 11.0, 12.4, 13.4, 13.7, 15.6, 16.0 and 17.6, or
(6) 6.7, 10.4, 11.0, 12.4,13.4, 13.7, 15.6, 16.0, 16.1, 17.6, 18.0, 18.6, 18.9, 20.1, 20.9 and 23.4.

## Example 2

***N*-{5-Chloro-2-[((2*S*)-3-{[1-(4-chlorobenzyl)piperidin-4-yl]amino}-2-hydroxy-2-methylpropyl)oxy]-4-hydroxyphenyl} propaneamide di-trifluoroacetate.**

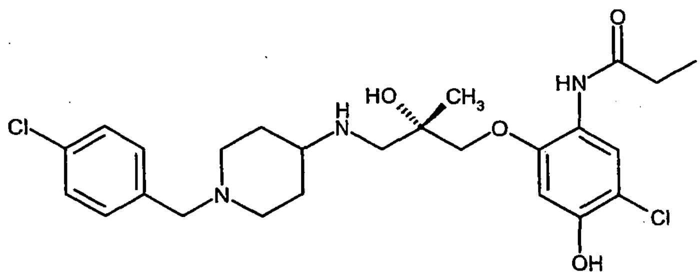

(i) *N*-(2-Hydroxy-4-methoxyphenyl)propanamide

To an ice-cooled solution of 2-hydroxy-4-methoxyaniline.HCl (600 mg, 3.4 mmol) and triethylamine (3 eq) in dichloromethane (25 mL) propionic anhydride (1.1 eq) was added dropwise. The solution was left at ambient temperature for 5 h. The reaction was quenched with water, the layers separated and the organic phase extracted with IN NaOH (aq) (3 x 25 mL). The pH of the aqueous phase was adjusted with concentrated HC1 to 5 and extracted with dichloromethane (3 x 25 mL). The organic phase was dried over anhydrous sodium sulphate, filtered and removed *in vacuo,* providing the subtitled compound as a brown solid (555 mg, 83%).

$^1$H NMR (300 MHz, $CDCl_3$-$d_6$) δ 7.04 (b), 6.83 (d, *J* = 8.4, 1H), 6.58 (d, *J* = 2.8, 1H), 6.43 (dd, $J_1$ = 8.4, $J_2$ = 2.8, 1H), 3.77 (s, 3H), 2.49 (q, *J* = 7.6, 2H), 1.29 (t, *J* = 7.5, 3H); APCI-MS: m/z 196 [$MH^+$].

(ii) *N*-(5-Chloro-2-hydroxy-4-methoxyphenyl)propanamide

To an ice-cooled solution ofN-(2-hydroxy-4-methoxyphenyl)propanamide (500 mg, 2.6 mmol) and dimethylformamide hydrogen chloride (1 eq) in DMF (5 mL), MCPBA (70%, 1 eq) was added in small portions. The reaction was stirred for an additional 5 minutes, afterwhich it was quenched with 1M $NaHCO_3$ (aq) (50 mL). The aquous phase was washed with ethyl acetate (50 mL): The organic phase was washed with water (3 x 25 mL), dried and removed *in vacuo,* providing the subtitled compound as a purple solid (408 mg, 71%).

$^1$H NMR (300 MHz, acetone-$d_6$) δ 9.68 (b, 1H), 9.12 (b, 1H), 7.37 (s, 1H), 6.62 (s, 1H), 3.83 (s, 3H), 2.49 (q, *J* = 7.7, 2H), 1.18 (t, *J* = 7.5, 3H); APCI-MS: m/z 229 [$M^+$].

(iii) *N*-(5-Chloro-4-methoy-2-{[(2*S*)-methyloxiran-2-yl]methoxy}lphenyl)prgpanamide

A suspension of *N*-(5-Chloro-2-hydroxy-4-methoxyphenyl)propanamide (202 mg, 0.88 nmol), [(2*S*)-2-methyloxiran-2-yl]methyl 3-nitrobenzenesulfonate (1 eq) and cesium carbonate (1.2 eq) in DMF (4 mL) was stirred at room temperature for 4 h. The mixture was separated over water (50 mL) and ethyl acetate (50 mL). The organic phase was washed with water (2 x 30 mL), dried and removed *in vacuo,* providing the subtitled compound as an off-white solid (249 mg, 95%)

$^1$H NMR (300 MHz, $CDC1_3$) 6 8.43 (s, 1H), 7.80 (b, 1H), 6.61 (s, 1H), 4.14 (m, 1H), 3.98 (m, 1H), 3.85 (s, 3H), 2.94 (m, 1H), 2.79 (m, 1H), 2.42 (q, *J* = 7.6,2H), 1.47 (s, 3H), 1.25 (t, *J* = 7.5, 3H); APCI-MS: m/z 299 [$MH^+$].

(iv) *N*-{5-Chloro-2-[((2*S*)-3-{[1-(4-chlorobenzyl)piperidin-4-yl]amino}-2-hydroxy-2-methylpropyl)oxy]-4-hydroxyphenyl} propaneamide di-trifluoroacetate

To a solution of 1-(4-chlorobenzyl)-piperidin-4-yl amine (50 mg, 0.2 mmol) and *N*-(5-chloro-4-methoxy-2-{[(2*S*)-methyloxiran-2-yl]methoxy}phenyl)propanamide (1 eq) in acetonitrile (5 mL), lithium perchlorate (10 eq) was added. The reaction mixture was refluxed for 18h. The reaction mixture was poured over a MEGA BE-SCX column (Bond Slut®, 5 g, 20 mL). The column was first washed with methanol (3x10 mL) and subsequently with a mixture of ammonia/ methanol (1/20, 3 x 10 mL). The basic layers were pooled and the solvent removed *in vacuo,* providing *N*-{5-chloro-2-[((2*S*)-3-{[1-(4-chlorobenzyl)piperidin-4-yl]amino}-2-hydroxypropyl)oxy]-4-methoxyphenyl} propaneamide as a light brown oil (100 mg, 86%), which was redissolved in dichlormethane (4 mL). The solution was cooled to 0 °C and 1M $BBr_3$ in dichloromethane (1 mL) added dropwise. The reaction was stirred for 18h, afterwhich it was quenched with methanol. The solvent was removed *in vacuo* and the residue purified by reverse phase prep. HPLC, using acetonitrile and water containing 0.1% TFA in gradient as mobile phase. Pooled fractions were freeze-dried to give the titled product as an amorphous white solid (38 mg, 39%).

$^1$H NMR (300 MHz, acetone-$d_6$) δ 8.66 (broad), 8.09 (3, 1H), 7.60 (d, *J*=8.4, 4H), 7.47 (d, *J* = 8.4, 4H), 6.78 (s, 1H), 4.41 (s, 2H), 4.10-3.93 (m, 2H), 3.70-3.65 (m, 4H), 3.44-2.39 (m, 1H), 3.20 (m, 2H), 2.52-2.37 (m, 6H), 1.38 (s, 3H), 1.10 (t, *J*=7.5, 3H); APCI-MS: m/z 510 [$MH^+$].

**Example 3**

***N*-{Chloro-2-[((2*S*)-3-{[1-(4-chlorobenzyl)piperidin-4-yl]amino}-2-hydroxy-2-methylpropyl)oxy]-4-hydroxyphenyl}acetamide di-trifluoroacetate.**

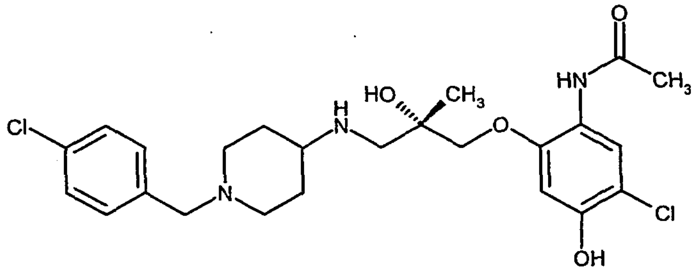

Synthesis analogous to that described for example 2 but wherein 2-hydroxy-4-methoxyaniline.HCl is reacted with acetic anhydride (1.1 eq).

$^{1}$H NMR (300 MHz, acetone-$d_6$) δ 8.77 (s, 1H), 8.06 (s, 1H), 7.61 (d, *J* = 8.2 Hz, 2H), 7.47 (d, *J* = 8.6 Hz, 2H), 6.79 (s, 1H), 4.43 (s, 2H), 4.08 (d, *J* = 9.9 Hz, 1H), 3.94 (d, *J* = 9.9 Hz, 1H), 3.79-3.61 (m, 3H), 3.68 (d, *J* = 12.5 Hz, 1H), 3.42 (d, *J* = 12.7 Hz,1H), 3.32-3.13 (m, 2H), 2.63-2.48 (m, 2H), 2.49-2.29 (m, 2H), 2.08 (s, 3H), 1.38 (s, 3H); APCI-MS: m/z 496 [M$^{+}$].

## Example 4

***N*-{5-Chloro-2-[((2*S*)-3-{[1-(4-chlorobenzyl)piperidin-4-yl]amino}-2-hydroxypropyl)oxy]-4-hydroxyphenyl} acetamide di-trifluoroacetate.**

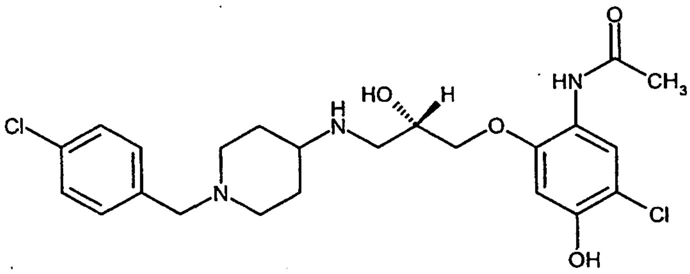

Synthesis analogous to that described for example 3 but wherein *N*-(5-chloro-2-hydroxy-4-methoxyphenyl) acetamide is reacted with S-(+)-glycidyl nosylate (1 eq)

$^{1}$H NMR (300 MHz, acetone-$d_6$) δ 8.64 (broad, NH), 8.21 (s, 1H), 7.59 (d, J = 9.0 Hz, 2H), 7.47 (d, J = 9.0 Hz, 2H), 6.74 (s, 1H), 4.41-4.35 (m, 3H), 4.13-4.01 (m, 2H), 3.69-3.40 (m, 5H), 3.14 (m, 2H), 2.55-2.47 (m, 2H), 2.31 (m, 2H), 2.09 (s, 3H); APCI-MS: m/z 482 [MH$^{+}$].

## Example 5

***N*-{5-Chloro-2-[((2*S*)-3-{[1-(4-chlorobenzyl)piperidin-4-yl]amino}-2-hydroxylpropyl)oxyl-4-hydroxyphenyl} propaneamide di-trifluoroacetate.**

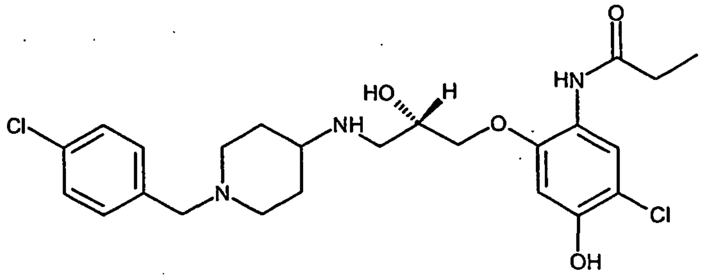

Synthesis analogous to that described for example 2 but wherein *N*-(5-chloro-2-hydroxy-4-methoxyphenyl) acetamide is reacted with S-(+)-glycidyl nosylate (1 eq)

$^1$H NMR (300 MHz, DMSO-$d_6$) δ 10.05 (broad), 9.78 (broad), 9.79 (broad), 9.00 (broad), 8.88 (broad), 7.79 (m, 1H), 7.62-7.50 (m, 4H), 6.63 (s, 1H), 5.98 (broad), 4.29 (m, 2H), 4.16 (m, 1H), 3.95-3.88 (m, 2H), 341-2.97 (m, 7H), 2.35-2.22 (m, 4H), 1.82-1.75 (m, 2H), 1.07 (m, 3H); APCI-MS: m/z 496 [MH$^+$].

__Human CCR1 binding assay__

**Membranes**

HEK293 cells, from ECACC, stably expressing recombinant human CCR1 (HEK-CCR1) were used to prepare cell membranes containing CCR1. The membranes were stored at -70°C. The concentration of membranes of each batch was adjusted to 10% specific binding of 33 pM [$^{125}$I] MIP-1α.

**Binding assay**

100 μL of HEK-CCR1 membranes diluted in assay buffer pH 7.4 (137 mM NaCl (Merck, Cat No 1.06404), 5.7 mM Glucose (Sigma, Cat No G5400), 2.7 mM KCl (Sigma, Cat No P-9333), 0.36 mM $NaH_2PO_4$ x $H_2O$ (Merck, Cat No 1.06346), 10 mM HEPES (Sigma, Cat No H3375), 0.1% (w/v) Gelatine (Sigma, Cat No G2625)) with the addition of 17500 units/L Bacitracin (Sigma, Cat No B1025) were added to each well of the 96 well filter plate (0.45 μm opaque Millipore cat no MHVB N4550). 12 μL of compound in assay buffer, containing 10% DMSO, was added to give final compound concentrations of $1x10^{-5.5}$-$1X10^{-9.5}$ M. 12 μl cold human recombinant MIP-1α (270-LD-050, R&D Systems, Oxford, UK), 10 nM final concentration in assay buffer supplemented with 10% DMSO, was included in certain wells (without compound) as non-specific binding control (NSB). 12 μl assay buffer with 10% DMSO was added to certain wells (without compound) to detect maximal binding (B0).

12 μL [$^{125}$I] MIP-1α, diluted in assay buffer to a final concentration in the wells of 33 pM, was added to all wells. The plates with lid were then incubated for 1.5 hrs at room temperature. After incubation the wells were emptied by vacuum filtration (MultiScreen Resist Vacuum Manifold system, Millipore) and washed once with 200 μl assay buffer. After the wash, all wells received an addition of 50 μL of scintillation fluid (OptiPhase "Supermix", Wallac Oy, Turko, Finland). Bound [$^{125}$I] MIP-1α was measured using a Wallac Trilux 1450 MicroBeta counter. Window settings: Low 5-High 1020, 1-minute counting/well.

**Calculation of percent displacement and IC$_{50}$**

The following equation was used to calculate percent displacement.

$$\text{Percent displacement} = 1 - ((\text{cpm test} - \text{cpm NSB}) / (\text{cpm B0} - \text{cpm NSB}))$$

where:

__cpm test__ = average cpm in duplicate wells with membranes and compound and [$^{125}$I] MIP-1α cpm;
__NSB =__ average cpm in the wells with membranes and MIP-1α and [$^{125}$I] MIP-1α (non-specific binding) cpm;
__B0__ = average cpm in wells with membranes and assay buffer and [$^{125}$I] MIP-1α (maximum binding).

The molar concentration of compound producing 50% displacement ($IC_{50}$) was derived using the Excel-based program XLfit (version 2.0.9) to fit data to a 4-parameter logistics function.
All compounds of the Examples 1 to 5 had $IC_{50}$ values of less than 20 nM.

**Inflammatory cell influx experiment in LPS-chaUenged rats**

The effect of a CCR1 receptor antagonist (*N*-{2-[((2*S*)-3-{[1-(4-chlorobenzyl)piperidin-4-yl]amino}-2-hydroxy-2-methylpropyl)oxy]-4-hydroxyphenyl}acetamide hemi-fumarate (2:1 salt), referred to here as Compound A) and budesonide, and their combination, on inflammatory cell influx was assayed by monitoring the effect on total cell number in broncholalveolar lavage (BAL) fluid of rats challenged intra-tracheally (i.t.) with Lipopolyaccharide (LPS) [N = 10 rats per treatment group].

Methodology

*LPS instillation:* Rats were anaesthetized with Efrane and put in a supine position, head up, on a board tilted at 30°. LPS (Lipopolysaccharide B.E.coli 026:B6) (2.5 μg/ml) dissolved in saline (0.9% NaCl), or saline alone (negative control) in a volume of 200 μl was administered i.t. using a modified metal cannula. Rats remained in this position until regaining consciousness.
*Preparation of solutions:* The homogenised budesonide was dissolved in ethanol and then mixed with 0.9% NaCl solution giving a final concentration of 0.002 mg budesonide /ml. Compound A was dissolved in 0.9% NaCl solution to a final concentration of 0.034 mg compound A.
Budesonide/compound A mixed formulations were made by dissolving compound A in the budesonide suspensions, giving a final concentration of 0.034 mg compound A /ml and 0.002 mg budesonide /ml.
*Treatments:* Animals were intratracheally instilled with solutions (1 ml/kg) of budesonide/compound A (0.002/ 0.034 mg/kg), or of budesonide (0.002 mg/kg) alone, or compound A (0.34 mg/kg) alone, or with saline (negative and positive control animals). The treatments were carried out under light anaesthesia (Efrane) to secure that the solution reached the lungs. The drugs were administrated 30 min before the LPS instillation.
*Termination:* 4 hours after the LPS challenge, rats were intraperitoneally injected with the mixture (0.3 ml) of pentobarbital (60 mg/ml, Apoteksbolaget, Sweden) and PBS (1:1) for 1 - 2 min.
*Bronchoalveolar lavage:* After termination, BAL was performed twice with PBS. The BAL fluid was centrifuged and the cell pellet was resuspended in PBS. The total numbers of BAL cells were counted in a SYSMEX cell counter.
The results of the experiment are shown in Figure 1. In Figure 1 "Saline / saline" rats represents the negative control rats treated with saline and challenged with saline. "Saline / LPS" animals represent the positive control rats treated with saline and challenged with LPS. The remaining three groups were all treated with the specified drugs and challenged with LPS.

## Claims

**1.** A pharmaceutical product comprising, in combination,

(a) a first active ingredient which is a compound of general formula

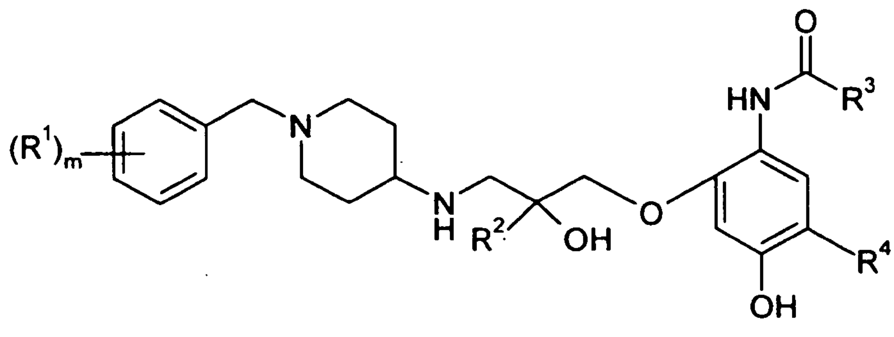

(I)

wherein

m is 0, 1 or 2;
each $R^1$ independently represents halogen;
$R^2$ represents a hydrogen atom or methyl;
$R^3$ represents the group $C_1$-$C_4$ alkyl;
and
$R^4$ represents hydrogen or halogen;

or a pharmaceutically acceptable salt thereof; and
(b) a second active ingredient which is a glucocorticosteroid.

**2.** A product according to claim 1, wherein $R^4$ is hydrogen or chlorine.

**3.** A product according to any preceding claim, wherein $R^3$ is methyl or ethyl.

**4.** A product according to any preceding claim, wherein the first active ingredient is selected from
*N*-{2-[((2*S*)-3-{([1-(4-chlorobenzyl)piperidin-4-yl]amino}-2-hydroxy-2-methylpropyl)oxy]-4-hydroxyphenyl}acetamide;
*N*-{5-Chloro-2-[((2*S*)-3-{[1-(4-chlorobenzyl)piperidin-4-yl]amino}-2-hydroxy-2-methylpropyl)oxy]-4-hydroxyphenyl}acetamide;
*N*-{5-Chloro-2-[((2*S*)-3-{[1-(4-chlorobenzyl)piperidin-4-yl]amino}-2-hydroxypropyl)oxy]-4-hydroxyphenyl}acetamide,
*N*-{5-Chloro-2-[((2*S*)-3-{[1-(4-chlorobenzyl)piperidin-4-yl]amino}-2-hydroxypropyl)oxy]-4-hydroxyphenyl}propaneamide, or
*N*-{5-Chloro-2-[((2*S*)-3-{[-1-(4-chlorobenzyl)piperidin-4-yl]amino}-2-hydroxy-2-mehylpropyl)oxy]-4-hydroxyphenyl}propaneamide,
or a pharmaceutically acceptable salt thereof.

**5.** A product according to any preceding claim, wherein the first active ingredient is a salt selected from the benzoate, fuorate or hemi-fumarate salt of *N*-{2-[((2*S*)-3-{[1-(4-chlorobenzyl)piperidin-4-yl]amino}-2-hydroxy-2-methylpropyl)oxy]-4-hydroxyphenyl}acetamide.

**6.** A product according to any preceding claim, wherein the glucocorticosteroid is budesonide.

**7.** A product according to any preceding claim, which further comprises a third active ingredient which is a bronchodilator.

**8.** A product according to claim 7, wherein the third active ingredient is a $\beta_2$-agonist.

**9.** A product according to claim 8, in which the third active ingredient is selected from formoterol or indacaterol.

**10.** A product according to any preceding claim, which is in a form suitable for administration by inhalation.

**11.** A product according to any preceding claim for use in therapy.

**12.** Use of a product according to any preceding claim, in the manufacture of a medicament for the treatment of a respiratory disease.

**13.** Use according to claim 12, wherein the respiratory disease is chronic obstructive pulmonary disease or asthma.

**14.** A kit comprising a preparation of a first active ingredient which is a compound of formula (I) as defined in any one of claims 1 to 4, or a pharmaceutically acceptable salt thereof, and a preparation of a second active ingredient which is a glucocorticosteroid and optionally instructions for the simultaneous, sequential or separate administration of the preparations to a patient in need thereof.

**15.** A pharmaceutical composition comprising, in admixture, a first active ingredient which is a compound of formula (I) as defined in any one of claims 1 to 4, or a pharmaceutically acceptable salt thereof, and a second active ingredient which is a glucocorticosteroid.

## Patentansprüche

1. Pharmazeutisches Produkt, enthaltend, in Kombination

(a) einen ersten Wirkstoff, bei dem es sich um eine Verbindung der allgemeinen Formel

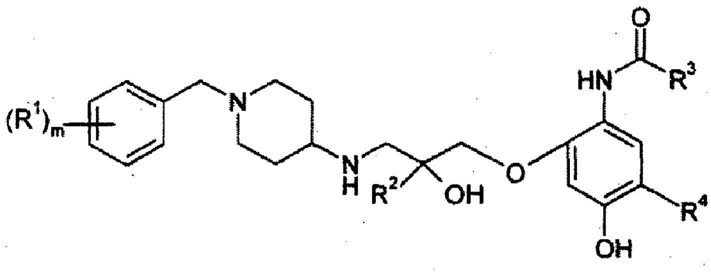

(I)

wobei

m für 0, 1 oder 2 steht;
$R^1$ jeweils unabhängig für Halogen steht;
$R^2$ für ein Wasserstoffatom oder Methyl steht;
$R^3$ für die Gruppe $C_1$-$C_4$-Alkyl steht;

und

$R^4$ für Wasserstoff oder Halogen steht;

oder ein pharmazeutisch annehmbares Salz davon; und
(b) einen zweiten Wirkstoff, bei dem es sich um ein Glucocorticosteroid handelt.

2. Produkt nach Anspruch 1, wobei $R^4$ für Wasserstoff oder Chlor steht.

3. Produkt nach einem der vorhergehenden Ansprüche, wobei $R^3$ für Methyl oder Ethyl steht.

4. Produkt nach einem der vorhergehenden Ansprüche, wobei der erste Wirkstoff ausgewählt ist aus
*N*-{2-[((2S)-3-{[1-(4-Chlorbenzyl)piperidin-4-yl]amino}-2-hydroxy-2-methylpropyl)oxy]-4-hydroxyphenyl}acetamid;
*N*-{5-Chlor-2-[((2S)-3-{[1-(4-chlorbenzyl)piperidin-4-yl]amino}-2-hydroxy-2-methylpropyl)oxy]-4-hydroxyphenyl}acetamid;
*N*-{5-Chlor-2-[((2S)-3-{[1-(4-chlorbenzyl)piperidin-4-yl]amino}-2-hydroxypropyl)oxy]-4-hydroxyphenyl}acetamid;
*N*-{5-Chlor-2-[((2S)-3-{[1-(4-chlorbenzyl)piperidin-4-yl]amino}-2-hydroxypropyl)oxy]-4-hydroxyphenyl}propanamid oder
*N*-{5-Chlor-2-[((2S)-3-{[1-(4-chlorbenzyl)piperidin-4-yl]amino}-2-hydroxy-2-methylpropyl)oxy]-4-hydroxyphenyl}propanamid oder ein pharmazeutisch annehmbares Salz davon.

5. Produkt nach einem der vorhergehenden Ansprüche, wobei es sich bei dem ersten Wirkstoff um ein Salz ausgewählt aus dem Benzoat-, Fuorat- oder Hemifumaratsalz von *N*-{2-[((2S)-3-{[1-(4-Chlorbenzyl)piperidin-4-yl]amino}-2-hydroxy-2-methylpropyl)oxy]-4-hydroxyphenyl}acetamid handelt.

6. Produkt nach einem der vorhergehenden Ansprüche, wobei es sich bei dem Glucocorticosteroid um Budesonid handelt.

7. Produkt nach einem der vorhergehenden Ansprüche, welches weiterhin einen dritten Wirkstoff enthält, bei dem es sich um einen Bronchodilator handelt.

8. Produkt nach Anspruch 7, wobei es sich bei dem dritten Wirkstoff um einen $\beta_2$-Agonisten handelt.

**9.** Produkt nach Anspruch 8, wobei der dritte Wirkstoff ausgewählt ist aus Formoterol oder Indacaterol.

**10.** Produkt nach einem der vorhergehenden Ansprüche, welches sich in einer für die Verabreichung durch Inhalation geeigneten Form befindet.

**11.** Produkt nach einem der vorhergehenden Ansprüche zur Verwendung in der Therapie.

**12.** Verwendung eines Produkts nach einem der vorhergehenden Ansprüche bei der Herstellung eines Medikaments zur Behandlung einer Atemwegserkrankung.

**13.** Verwendung nach Anspruch 12, wobei es sich bei der Atemwegserkrankung um chronische obstruktive Atemwegserkrankung oder Asthma handelt.

**14.** Kit, enthaltend, eine Zubereitung eines ersten Wirkstoffs, bei dem es sich um eine wie in einem der Ansprüche 1 bis 4 definierte Verbindung der Formel (I) oder ein pharmazeutisch annehmbares Salz davon handelt, und eine Zubereitung eines zweiten Wirkstoffs, bei dem es sich um ein Glucocorticosteroid handelt, und gegebenenfalls Anleitungen für die gleichzeitige, aufeinanderfolgende oder getrennte Verabreichung der Zubereitungen an einen derer bedürftigen Patienten.

**15.** Pharmazeutische Zusammensetzung enthaltend, als Mischung, einen ersten Wirkstoff, bei dem es sich um eine wie in einem der Ansprüche 1 bis 4 definierte Verbindung der Formel (I) oder ein pharmazeutisch annehmbares Salz davon handelt, und einen zweiten Wirkstoff, bei dem es sich um ein Glucocorticosteroid handelt.

## Revendications

**1.** Produit pharmaceutique comprenant, en combinaison,

(a) un premier principe actif qui est un composé de formule générale

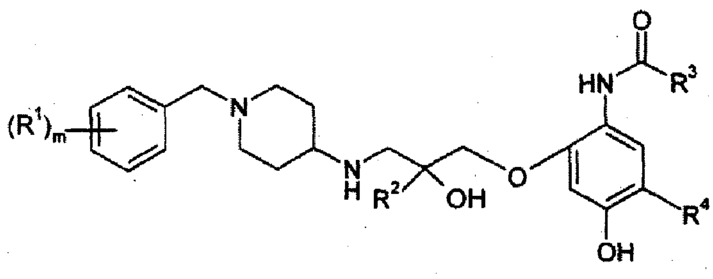

(I)

dans laquelle

m est 0, 1 ou 2 ;
chaque $R^1$ représente indépendamment halogène ;
$R^2$ représente un atome d'hydrogène ou méthyle ;
$R^3$ représente un groupement alkyle en $C_1$-$C_4$ ;
et
$R^4$ représente hydrogène ou halogène ;

ou un sel pharmaceutiquement acceptable de celui-ci ; et
(b) un deuxième principe actif qui est un glucocorticostéroïde.

**2.** Produit selon la revendication 1, **caractérisé en ce que** $R^4$ est hydrogène ou chlore.

**3.** Produit selon l'une ou l'autre des revendications précédentes, **caractérisé en ce que** $R^3$ est méthyle ou éthyle.

4. Produit selon l'une quelconque des revendications précédentes, **caractérisé en ce que** le premier principe actif est choisi parmi
le *N*-{2-[((2S)-3-{[1-(4-chlorobenzyl)pipéridin-4-yl]amino}-2-hydroxy-2-méthylpropyl)oxy]-4-hydroxyphényl}acétamide ;
le *N*-{5-chloro-2-[((2S)-3-{[1-(4-chlorobenzyl)pipéridin-4-yl]amino}-2-hydroxy-2-méthylpropyl)oxy]-4-hydroxyphényl}acétamide ;
le *N*-{5-chloro-2-[((2S)-3-{[1-(4-chlorobenzyl)pipéridin-4-yl]amino}-2-hydroxypropyl)oxy]-4-hydroxyphényl}acétamide ;
le *N*-{5-chloro-2-[((2S)-3-{[1-(4-chlorobenzyl)pipéridin-4-yl]amino}-2-hydroxypropyl)oxy]-4-hydroxyphényl}propanamide ou
le *N*-{5-chloro-2-[((2S)-3-{[1-(4-chlorobenzyl)pipéridin-4-yl]amino}-2-hydroxy-2-méthylpropyl)oxy]-4-hydroxyphényl}propanamide,
ou un sel pharmaceutiquement acceptable de ceux-ci.

5. Produit selon l'une quelconque des revendications précédentes, **caractérisé en ce que** le premier principe actif est un sel choisi parmi le sel de benzoate, de fuorate ou d'hémi-fumarate de *N*-{2-[((2S)-3-{[1-(4-chlorobenzyl) pipéridin-4-yl]amino}-2-hydroxy-2-méthylpropyl)oxy]-4-hydroxyphényl}acétamide.

6. Produit selon l'une quelconque des revendications précédentes, **caractérisé en ce que** le glucocorticostéroïde est le budésonide.

7. Produit selon l'une quelconque des revendications précédentes, **caractérisé en ce qu'**il comprend en outre un troisième principe actif qui est un bronchodilatateur.

8. Produit selon la revendication 7, **caractérisé en ce que** le troisième principe actif est un $\beta_2$-agoniste.

9. Produit selon la revendication 8, **caractérisé en ce que** le troisième principe actif est choisi parmi le formotérol ou l'indacatérol.

10. Produit selon l'une quelconque des revendications précédentes, **caractérisé en ce qu'**il est sous une forme adaptée à l'administration par inhalation.

11. Produit selon l'une quelconque des revendications précédentes, destiné à être utilisé en thérapie.

12. Utilisation d'un produit selon l'une quelconque des revendications précédentes, dans la fabrication d'un médicament destiné au traitement d'une maladie respiratoire.

13. Utilisation selon la revendication 12, **caractérisée en ce que** la maladie respiratoire est la bronchopneupathie chronique obstructive ou l'asthme.

14. Kit comprenant une préparation d'un premier principe actif qui est un composé de formule (I) tel que défini dans l'une quelconque des revendications 1 à 4, ou un sel pharmaceutiquement acceptable de celui-ci, et une préparation d'un deuxième principe actif qui est un glucocorticostéroïde et éventuellement les instructions pour l'administration simultanée, séquentielle ou séparée des préparations à un patient en ayant besoin.

15. Composition pharmaceutique comprenant, en mélange, un premier principe actif qui est un composé de formule (I) tel que défini dans l'une quelconque des revendications 1 à 4, ou un sel pharmaceutiquement acceptable de celui-ci, et un deuxième principe actif qui est un glucocorticostéroïde.

Fig. 1

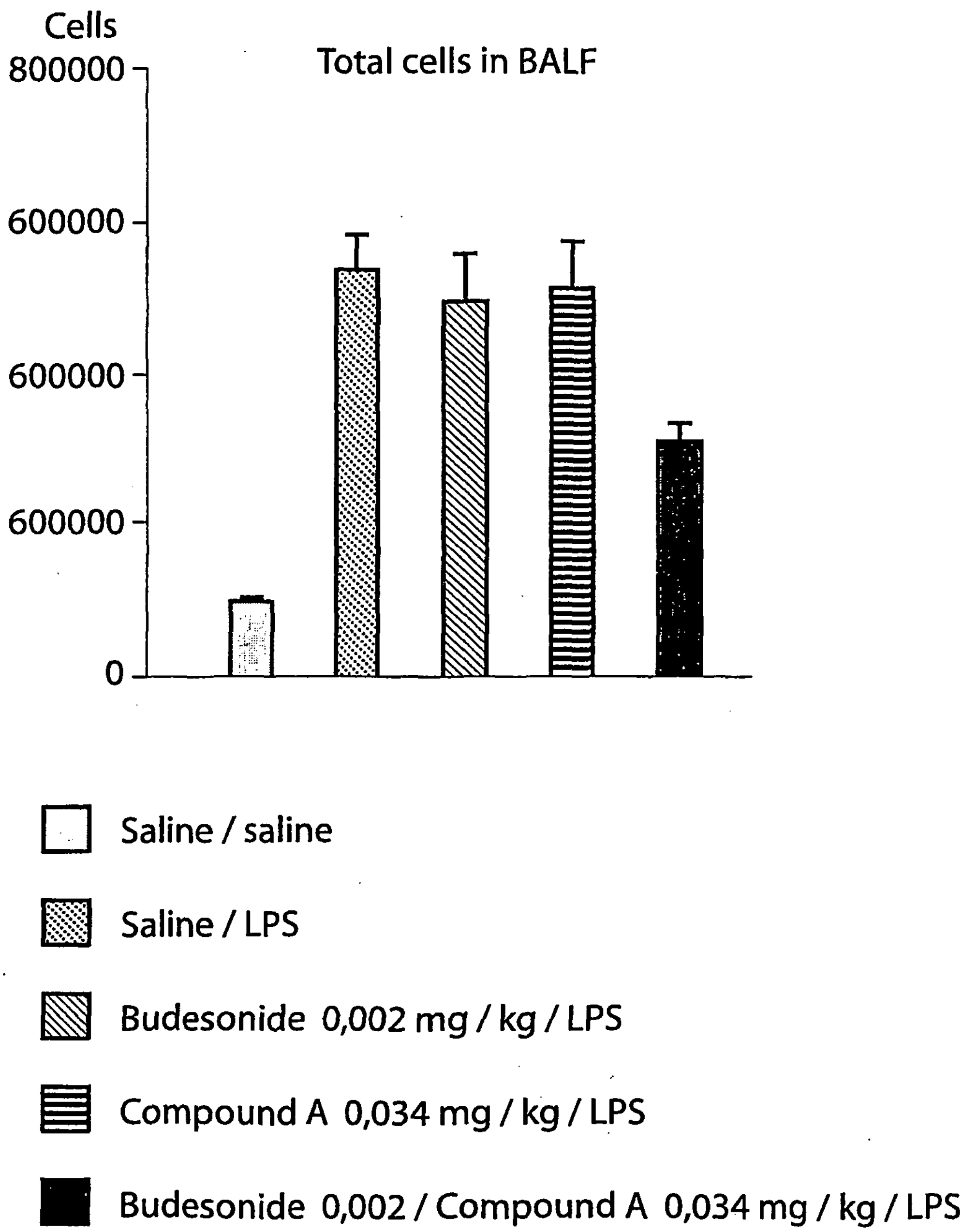
Cells
Total cells in BALF
800000
600000
600000
600000
0
Saline / saline
Saline / LPS
Budesonide 0,002 mg / kg / LPS
Compound A 0,034 mg / kg / LPS
Budesonide 0,002 / Compound A 0,034 mg / kg / LPS

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0198273 A
- WO 03051839 A
- WO 200212265 A
- WO 200212266 A
- WO 200288167 A
- DE 4129535
- WO 200200679 A
- WO 2005041980 A
- DE 2323215
- US 3929768 A
- US 4579854 A
- WO 200276933 A
- WO 2003042164 A
- WO 2005025555 A
- WO 2004032921 A
- US 20030055080 A
- WO 9205147 A


### Non-patent literature cited in the description


- **Tomlinson et al.** *Thorax,* 2005, vol. 60, 282-287
- United States Pharmacopeial. United States Pharmacopeial Convention, 2002, 2088-2089
- *Arzneimittel-Forschung,* 1979, vol. 29 (11), 1687-1690
- Fundamentals of Crystallography. Oxford University Press, 1992
- Introduction to X-Ray Powder Diffractometry. John Wiley & Sons, 1996
- Chemical Crystallography. Clarendon Press, 1948
- X-ray Diffraction Procedures. John Wiley & Sons, 1974
- United States Pharmacopeia. United States Pharmacopeial Convention, 2002, 2088-2089